# NEW EUROPEAN PATENT SPECIFICATION

(11) **EP 0 957 872 B2**
(45) Date of publication and mention of the opposition decision: **24.10.2007**
(45) Mention of the grant of the patent: 29.10.2003
(21) Application number: 97953178.7
(22) Date of filing: 15.12.1997
(51) Int. Cl.: A61F 13/15

(54) **DIAPER WITH PLEATS FOR CONTAINMENT OF LIQUID AND SOLID WASTE**
WINDEL MIT FALTEN ZUR EINDÄMMUNG VON FLÜSSIGEN UND FESTEN ABFALLSTOFFEN
COUCHE A FRONCES CONCUE POUR LA RETENTION DES REJETS LIQUIDES ET SOLIDES

(30) Priority: 23.12.1996 US 779990; 02.07.1997 US 886950
(43) Date of publication of application: 24.11.1999
(73) Proprietor: KIMBERLY-CLARK WORLDWIDE, INC., Neenah, Wisconsin 54956 (US)
(72) Inventor: PUTZER, Melissa, C., Oshkosh, WI 54904 (US); SCHLINZ, Daniel, R., Greenville, WI 54942 (US); ABUTO, Frank, P., Duluth, GA 30097 (US)
(74) Representative: Davies, Christopher Robert
(86) International application number: PCT/US1997/022838
(87) International publication number: WO 1998/027907

(56) References cited:
- EP-A- 0 190 881
- EP-A- 0 681 820
- WO-A-94/10951
- WO-A-95/08972
- WO-A-97/16144
- GB-A- 2 182 841
- US-A- 3 180 335
- US-A- 4 695 278
- US-A- 4 909 803
- US-A- 5 527 303

## Description

The present invention relates to absorbent articles such as disposable diapers, and more particularly to absorbent articles that have a pleated backsheet to provide improved containment.

Infants and other incontinent individuals wear absorbent articles such as diapers to receive and contain urine and other body exudates. Such articles are intended to prevent body exudates from soiling, wetting, or otherwise contaminating clothing or other articles, such as bedding, that come in contact with the wearer. The most common mode of failure for such products occurs when body exudates leak out of the gaps between the absorbent article and the wearers leg or waist to adjacent clothing because they are not immediately absorbed within the article. This is most evident with loose fecal material, which is not easily absorbed by the absorbent article.

Several solutions to this problem have been proposed. For example, U.S. Pat. No. 4,490,148 describes an oblong absorbent body having lateral edge portions folded over to form side flaps. The edges of the side flaps contain elastic to contact the thigh creases of the crotch.

U.S. Pat. No. 5,527,303 shows an incontinent pad that has a backsheet with at least one pleat that is tacked with a soluble adhesive so that when the adhesive is wetted, the adhesive dissolves to allow the pleat to expand In conjunction with the absorbent core. A disadvantage to this absorbent article is that the liquid must first pass through the absorbent core before it enters the pleat, if at all. In addition, this absorbent article does not allow for the passage of solid matter Into the pleat EP-A-0190881 describes a disposable waste containment garment having side-edge, leakage-guard gutters having closed ends. GB-A-2182841 discloses an absorbent article having the features of the preamble of claim 1. The present invention is characterised over this disclosure by having at least one pleat that is unadhered in the intermediate region so that the pleat can expand away from the wearer's body.

Thus the present invention provides an absorbent article that has an expandable backsheet for collecting liquid and solid matter without it having to first pass through the absorbent core. Where the present invention is in the form of a diaper, a particular advantage is that the void volume of the diaper is increased at the outer surface of the diaper rather than internally, which does not increase the crotch width. Importantly, because the void volume is increased at the outer surface, the space between the wearer and the absorbent core increases with a concomitant reduction in discomfort to the wearer. In addition, the presence of pleats in accordance with the present invention allows the containment of both liquid and solid material without unduly increasing the overall bulk of the diaper.

The present invention provides a disposable absorbent article such as a diaper that is both thin and suitable for containing large volumes of liquid and solid body exudates without leakage. While the present invention will be particularly described in the form of a diaper, one of skill in the art will appreciate that it may be advantageously used with incontinent briefs, diaper holders, training pants, and the like. The diaper has a front waist region, a back waist region and an intermediate (or crotch) region that interconnects the front and back waist regions and is disposed between laterally opposed side margins. The diaper includes a liquid pervious liner suitable for contact with the wearer's body, a liquid impervious backsheet, and an absorbent core disposed between the liner and the backsheet. The liner may completely surround the absorbent core or may partially surround the portion of the absorbent core facing the wearer. In either case, the liner is preferably joined to the backsheet.

Each side or lateral margin defines a leg opening. An elastic member is connected to each side or lateral margin of the diaper to provide elasticized leg openings. A containment flap may be associated with the elastic member and is located inwardly or toward the central portion of the diaper to provide additional containment of body exudates.

The backsheet includes at least one pleat located adjacent and inwardly of each side margin said pleat being unadhered in the intermediate region so that it allows the backsheet to expand away from the wearer's body in the intermediate region such that the pleat defines an expanded volume when the pleat is expanded which is greater than the volume when the pleat is unexpanded. The pleat is accessible to fluid either by first passing through the absorbent core or by passing around the absorbent core. Preferably, each pleat extends substantially parallel to the longitudinal axis. More preferably, each pleat extends substantially the entire longitudinal distance from the front waist region to the rear waist region. In a particularly preferred embodiment, each pleat extends substantially the entire longitudinal distance from the front waist region to the rear waist region with the pleat being adhered in the front and rear waist regions.

A preferred embodiment of the present invention will now be described with reference to the accompanying drawings in which:
FIG. 1 is a perspective view of a diaper embodiment of the disposable absorbent article of the present invention with the surface of the diaper that contacts the wearer facing the viewer.
FIG. 2 is a cross sectional view of one embodiment of the article of FIG. 1 showing the pleat in a folded or closed state. In this embodiment, the liner partially surrounds the portion of the absorbent core facing the wearer's body and the absorbent core is located in the central portion of the diaper between the pleats.
FIG. 3 is a cross sectional view of one embodiment of the article of FIG. 2 showing the pleat in an open or expanded state.
FIG. 4 is a cross sectional view of another article, that is not in accordance with the present invention and is shown by way of illustration only, showing the pleat in an open or expanded state. The absorbent structure extends substantially the entire lateral dimension of the diaper.
FIG. 5 is a cross sectional view of another embodiment of the article of the present invention showing the pleat in an open or expanded state. In this embodiment, the liner completely surrounds the absorbent core.
FIG. 6 is a cross sectional view of another embodiment of the article of the present invention showing the pleat in the closed state. This embodiment is similar to that shown in FIG. 2 except that the liner does not extend into the pleat formed in the backsheet until the pleat is in the open or expanded state.

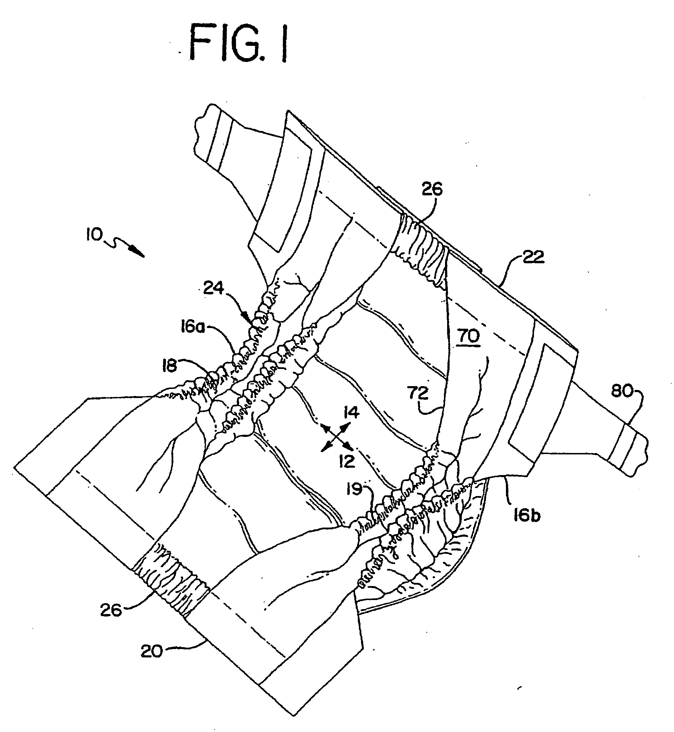

With reference to FIG. 1, an absorbent article, such as a diaper 10 is shown with the surface of the diaper that contacts the wearer facing the viewer. The diaper has a cross-wise, lateral axis or dimension 12 and a length-wise, longitudinal axis or dimension 14. The diaper 10 has laterally opposed side margins 16a, 16b, a front waistband region 20, a back waistband region 22, and an intermediate portion or crotch region 24 that interconnects the front and back waistbands and is laterally disposed between the side margins. The waistbands are arranged to encircle the front and back portions of the wearer's waist, and the intermediate portion is intended for placement between the wearer's legs. The waistbands may also have waist elastics 26.

The diaper comprises a substantially liquid-permeable liner 30, a liquid impervious backsheet layer 40, and an absorbent core 50 sandwiched between the liner and backsheet. The side margins define leg openings. As shown in FIG. 1, an elastic member 18 is provided in the region of each side margin to provide elasticized, gathered leg openings. A containment flap 19 may be associated with the elastic member. The liner, backsheet, absorbent structure, elastic members 18 and 26, and containment flaps 19 may be assembled in a variety of well-known diaper configurations. In accordance with the principles of the present invention, the backsheet is provided with at least one pleat adjacent each side margin.

The liner 30 presents a body-facing surface 32 which is compliant, soft-feeling, and non-irritating to the wearer's skin and a garment facing surface 34 associated with the absorbent structure. Preferably, the liner is sufficiently porous to be liquid permeable, permitting liquid to penetrate through its thickness. The liner is typically used to help isolate the wearer's skin from liquids held in the absorbent structure.

The liner fabrics may be composed of a substantially hydrophobic and substantially nonwettable material, and the hydrophobic material may optionally be treated with a surfactant or otherwise processed to impart a desired level of wettability and hydrophilicity. A suitable liner may be manufactured from a wide selection of web materials, such as porous foams, reticulated foams, apertured plastic films, natural fibers (for example, wood or cotton fibers), synthetic fibers (for example, polyester or polypropylene fibers), or a combination of natural and synthetic fibers. Various woven and nonwoven fabrics can be used for the liner. For example, the liner may be composed of a meltblown or spunbonded web of polyolefin fibers. The liner may also be a bonded-carded-web composed of natural and/or synthetic fibers.

An absorbent core, such as absorbent structure 50, is positioned between the liner 30 and backsheet 40 to form diaper 10. The absorbent core has a construction which is generally compressible, conformable, non-irritating to the wearer's skin, and capable of absorbing and retaining liquid body exudates. The absorbent structure may comprise a single, integral piece of material, or alternatively, may comprise a plurality of individual separate pieces of material which are operably assembled together. Where the absorbent structure comprises a single, substantially integral piece of material, the material could include the desired structural features formed into selected spatial regions thereof. Where the absorbent structure comprises multiple pieces, the pieces may be configured as discrete layers or as other nonlayered shapes and configurations. Furthermore, the individual pieces may be coextensive or non-coextensive, depending upon the requirements of the product. It is preferred, however, that each of the individual pieces be arranged in an operable, intimate contact along at least a portion of its boundary with at least one other adjacent piece of the absorbent structure. Preferably, each piece is connected to an adjacent portion of the absorbent structure by a suitable bonding and/or fiber entanglement mechanism, such as ultrasonic or adhesive bonding, or mechanical or hydraulic needling.

The absorbent structure may be manufactured in a wide variety of sizes and shapes (for example, rectangular, trapezoidal, T-shape, I-shape, hourglass shape, etc.) and from a wide variety of materials. Absorbent article structures suitable for use with the present invention are described for example in U.S. Pat No. 5,192,606. The size and the absorbent capacity of the absorbent structure should be compatible with the size of the intended wearer and the liquid loading imparted by the intended use of the absorbent article.

Various types of wettable, hydrophilic fibrous material can be used to form the component parts of the absorbent structure. Examples of suitable fibers include naturally occurring organic fibers composed of intrinsically wettable material, such as cellulosic fibers; synthetic fibers composed of cellulose or cellulose derivatives, such as rayon fibers; inorganic fibers composed of an inherently wettable material, such as glass fibers; synthetic fibers made form inherently wettable thermoplastic polymers, such as particular polyester or polyamide fibers; and synthetic fibers composed of a nonwettable thermoplastic polymer, such as polypropylene fibers, which have been hydrophilized by appropriate means. The fibers may be hydrophilized, for example, by treatment with silica, treatment with a material which has a suitable hydrophilic moiety and is not readily removable from the fiber, or by sheathing the nonwettable, hydrophobic fiber with a hydrophilic polymer during or after the formation of the fiber. For the purposes of the present invention, it is contemplated that selected blends of the various types of fibers mentioned above may also be employed.

The entire absorbent structure or any individual portion thereof, can be overwrapped in a hydrophilic high wet-strength envelope web, such as a high wet-strength tissue or a synthetic fibrous web. Such overwrapping web can also increase the in-use integrity of the absorbent structure. The web can be suitably bonded, such as with adhesive, to the absorbent structure and to other components of the product construction.

The backsheet 40 may be composed of a liquid permeable material, but preferably comprises a material which is configured to be substantially impermeable to liquids. For example, a typical backsheet can be manufactured from a thin plastic film, or other flexible liquid-impermeable material. As used in the present specification, the term "flexible" refers to materials which are compliant and which will readily conform to the general shape and contours of the wearer's body. The backsheet can help prevent the exudates contained in the absorbent structure from wetting articles such as bedsheets and overgarments which contact the diaper.

The backsheet may optionally be composed of a microporous, "breathable" material which permits vapors to escape from the absorbent structure while still preventing liquid exudates from passing through the backsheet. For example, the breathable backsheet may be composed of a microporous polymer film a nonwoven fabric which has been coated or otherwise treated to impart a desired level of liquid impermeability.

The size of the backsheet is typically determined by the size of the absorbent structure and the exact diaper design selected. The backsheet, for example, may have a generally T-shape, a generally I-shape or a modified hourglass shape, and may extend beyond the terminal edges of the absorbent structure by a selected distance. Preferably, the backsheet has an hourglass shape.

The liner and backsheet may be generally coextensive, and may have a length and width dimension that are generally larger than the corresponding dimensions of the absorbent structure. Alternatively, the liner may simply surround the absorbent structure while the backsheet has a length and width dimension larger than the corresponding dimensions of the absorbent structure.

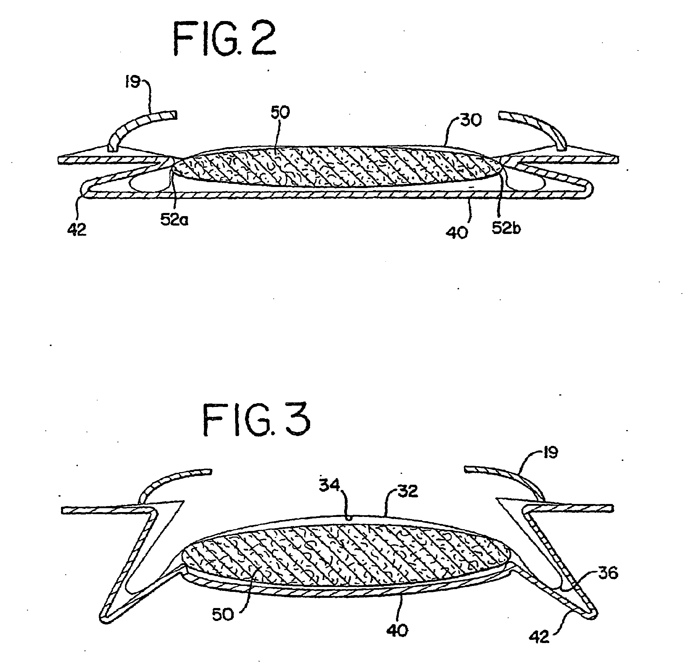

The liner 30 and backsheet 40 are connected or otherwise associated together in an operable manner. As used herein, the term "associated" encompasses configurations in which the liner is directly joined to the backsheet by affixing the liner directly to the backsheet and configurations wherein the liner is joined to the backsheet by affixing the liner to intermediate members which in turn are affixed to the backsheet. As best seen in FIG. 2, the liner and backsheet can be affixed directly to each other at the diaper periphery by attachment means (not shown) such as an adhesive, sonic bonds, thermal bonds or any other attachment means known in the art. For example, a uniform continuous layer of adhesive, a patterned layer of adhesive, a sprayed pattern of adhesive or an array of separate lines, swirls or spots of construction adhesive may be used to affix the liner to the backsheet.

It should be readily appreciated that the above-described attachment means may also be employed to interconnect and assemble together the various other component parts of the article described herein.

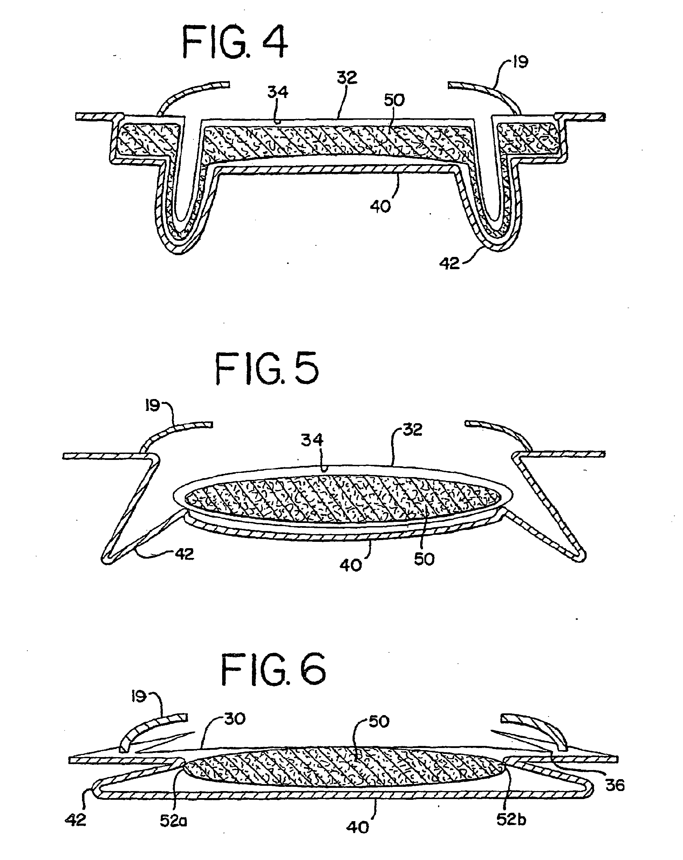

In one embodiment of the present invention best seen in FIG. 2, the liner 30 is disposed and secured in facing relation with the backsheet layer 40. The marginal side regions of the liner are operably connected to corresponding marginal side regions of the backsheet layer. Each of the attached marginal side regions of the liner and backsheet layers is located laterally outboard of the associated side edge region of the absorbent structure. In this embodiment, the liner partially surrounds the uppermost portion of the absorbent structure, i.e., the portion of the absorbent structure facing the wearer. In addition, in this embodiment, the liner contains a pleat 36 that extends into the pleat 42 formed in the backsheet. Alternatively, as shown in FIG. 6, the liner partially surrounds the uppermost portion of the absorbent structure but the liner pleat 36 does not extend into the backsheet pleat 42 when the pleat 42 is unexpanded. When the pleat 42 expands, the liner pleat 36 will assume the configuration shown in FIG. 3. The side edges of the absorbent structure 52a, 52b do not extend laterally beyond innermost portion of the pleat formed in the backsheet.

In addition, where the absorbent structure does not extend beyond the innermost portion of the pleat, filler material (not shown) may be incorporated in the pleated area sandwiched between the liner and the backsheet. The filler material may include tissue, wicking material, super-absorbent or chemical material that can absorb and/or mix with the body exudate to absorb, eliminate odor, or neutralize the body exudates.

In another arrangement as best shown in FIG. 4, the side edges of the absorbent structure may extend substantially from one side margin to the other side margin either along the entire longitudinal length of the diaper or only in the front and back waistband regions. In either case, it may be preferable if the absorbent structure has a smaller thickness in the area between the innermost portion of the pleat and the outermost portion of the pleat so that the bulk of the diaper is not unduly increased. This arrangement is not in accordance with the present invention and is shown by way of illustration only.

Alternatively, in another embodiment of the present invention best seen in FIG. 5, the liner completely surrounds the absorbent structure. In this embodiment, the liner is preferably associated with or joined to the backsheet. More preferably the liner is associated with the backsheet only in the area between the innermost portion of the pleats.

In accordance with the principles of the present invention and as will be more fully explained below, it is to be understood that in each of the above embodiments body exudates may access the pleats directly (by passing around the absorbent structure) or indirectly (by passing through the absorbent structure). At least one pleat 42 is provided in the backsheet adjacent each side margin. Alternatively, a plurality of pleats may be provided adjacent each side margin. Preferably, the pleats extend in a direction substantially parallel to the longitudinal axis of the diaper along the entire length or a portion of the length of the diaper. More preferably, the pleats extend along the entire length of the diaper. In this instance, the portion of the pleats located in the front and back waistband region are tacked or adhered so that they do not open, while in the intermediate or crotch region the pleats are unadhered. Thus, the pleats allow the backsheet to expand away from the wearer's body surface in the crotch region while maintaining a close fit in the front and back waistband regions and preventing any leakage in those areas.

Preferably, the pleat is located inwardly of the side margin and thus the elastic member. By providing the pleat in this location, the liquid and solid body exudates need not first pass through the absorbent structure before settling within the expanded volume defined by the expanded pleat. Referring to FIGs. 3 and 5, it will be appreciated that the body exudates may have a flow path through or around the absorbent structure.

The pleat may be formed in any suitable manner. For example, the liner and backsheet can be pre-pleated with folding boards using a machine or by hand. In this method, the diaper will assume a configuration substantially similar to that shown in FIG. 6. In yet another method, the liner and backsheet may be pleated after the absorbent core has been sandwiched by the liner and backsheet to obtain a diaper configuration shown in FIG. 2. Of course, other methods of providing pleats will be known to those of skill in the art.

An elastic member 18 is disposed along each of the longitudinal side edges 16a, 16b of the diaper. The elastic member 18 can be connected to either or both of the liner and backsheet layers to provide elasticized side margins of the diaper article, and can be arranged to draw and hold diaper 10 against the legs of the wearer. No matter where the elastic member is connected, it should be appreciated that in the intermediate region, the elastic member should be connected outwardly of the outermost portion of the pleat so that the pleat can fully expand. Waist elastic members 26 may also be disposed adjacent either or both of the end edges of diaper 10 to provide elasticized waistbands.

Preferably, the elastic members 18 and 26 are secured to the diaper in an elastically contractible condition so that in a normal, under strain configuration, the elastic members effectively contract against the diaper. For example, the elastic members may be stretched and secured while the diaper is in an uncontracted condition. Alternatively, the diaper may be contracted, for example, by pleating, and the elastic members secured and connected to the diaper while the elastic members are in their unrelaxed or unstretched condition. Still other means, such as heat-shrink elastic material, may be used to gather and shrink the garment.

The elastic members 18 may extend the entire length of the backsheet. Alternatively, the elastic members may extend essentially along the complete length of crotch region 24 or any other length suitable providing the arrangement of elastically contractible lines desired for the particular diaper design.

Elastic members 18 and 26 may have any of a multitude of configurations. For example, the width of the individual elastic members 18 may be varied from 0.25 millimeters (0.01 inches) to 25 millimeters (1.0 inches) or more. The elastic members may comprise several parallel or non-parallel strands of elastic material, or may be applied in a rectilinear or curvilinear arrangement. Where the strands are non-parallel, two or more of the strands may intersect or otherwise interconnect within the elastic member. The elastic members may be affixed to the diaper in any of several ways which are known in the art. For example, the elastic members may be ultrasonically bonded, heat and pressure sealed using a variety of bonding patterns, or adhesively bonded to the diaper with sprayed or swirled patterns of hot melt or other type of adhesive.

In the illustrated embodiments of the invention, the elastic member comprises a carrier sheet 70 to which are attached a grouped set of elastics composed of a plurality of individual elastic strands. The elastic strands may intersect or be interconnected, or be entirely separated from one another. The shown carrier sheet may, for example, comprise a 0.002 cm thick film of unembossed polypropylene material. The shown elastic strands can, for example, be composed of LYCRA elastomer available from DuPont. Each elastic strand is typically within the range of about 620-1050 decitex (dtx). In addition, the elastic strands may be generally straight or optionally curved.

As noted above, the diaper can include elasticized containment flaps associated with or connected to either or both of the liner and backsheet to provide an elasticized containment. As with the elastic member, the containment flap should, at least in the intermediate region, be connected outwardly of the outermost portion of the pleat so that the pleat can fully expand. In one embodiment, as shown in FIG. 1, the flaps 19 are provided as part of a side carrier 70. The containment flaps are located inboard the elastic members 18. The containment flaps may be constructed of a fibrous material which is similar to the material comprising the liner. Other conventional materials, such as polymer films, may also be used. In other aspects of the invention, the flaps are constructed of a material which is permeable to gas, such as ambient air. Alternative configurations of the invention can include barrier flaps which are constructed of a material which is resistant to a passage of aqueous liquid , such a urine, therethrough. For example, the flaps may be constructed of a spunbond-meltblown-spunbond (SMS) laminate material.

Each containment flap includes a movable edge region 72 that can include one or more individual strands of elastomeric material. For example, a plurality of elastic strands may be configured in a spatially separated, generally parallel arrangement, and a suitable elastic strand can, for example, be composed of a LYCRA elastomer. The elastic is preferably connected to the movable edge of the containment flap in an elastically contractible condition such that the contraction of the elastic components thereof gathers and shortens the edge of the containment flap. As a result, the movable edge of each containment flap tends to position itself in a spaced relation away from the bodyside surfaces of liner 32 toward a generally upright and approximately perpendicular configuration, especially in the crotch section of the diaper. By providing a generally upright and approximately perpendicular configuration, the flap operates in conjunction with the above-described pleats to provide a volume for the containment of liquid and solid body exudates.

Fastening means, such as tape tab fasteners 80, are typically applied to the back waistband region 22 of diaper 10 to provide a mechanism for holding the diaper on the wearer. Tape tab fasteners 80 can be any of those well known in the art, and are typically applied to the comers of diaper 10. For example, adhesive fasteners, mechanical fasteners, hook and loop fasteners, snaps, pins or buckles, may be used alone, or in combination. In the shown configuration, the fasteners are the male or hook portion of a hook-and-loop fastener, which are constructed to releasably adhere to a landing zone patch (not shown) attached to the front waistband section of the diaper to provide a refastenable fastening system. In the shown configuration, the landing zone patch comprises the female or loop portion of a hook-and-loop fastener.

It should be understood that a wide range of changes and modifications can be made to the embodiments described above. It is therefore intended that the foregoing description illustrates rather than limits this invention and that it is the following claims which define this invention.

## Claims

1. A disposable absorbent article (10) having a longitudinal axis (14) and a transverse axis (12), a front waistband region (20), a back waistband region (22), and an intermediate region (24) interconnecting the front and back waistband regions and disposed between laterally opposed side margins (16a,16b), the article further comprising:
a. a liquid pervious liner (30);
b. a liquid impervious backsheet (40);
c. an absorbent core (50) disposed between the liner and the backsheet, the absorbent core having an uninterrupted cross-section and a first (52a) and a second (52b) lateral side edge; **characterised by**
d. at least one pleat (42) formed in the backsheet adjacent each side margin, said pleat being unadhered in the intermediate region (24) so that it allows the backsheet (40) to expand away from the wearer's body in the intermediate region (22), the pleat defining an expanded volume when the pleat expands which is greater than the volume when the pleat is unexpanded and, when the pleat expands, the first and second lateral side edges of the absorbent core do not extend beyond the innermost portion of the pleat formed in the backsheet, and in that the disposable absorbent article (10) further comprises a containment flap (19) spaced inwardly from each side margin, and an elastic member (18) connected to each side margin (16a,16b) to provide elasticized leg openings.

2. The disposable absorbent article (10) of claim 1 wherein the at least one pleat (42) extends in a direction substantially parallel to the longitudinal axis (14).

3. The disposable absorbent article (10) of claim 2 wherein the at least one pleat (42) extends substantially from the front waistband region (20) to the back waistband region (22).

4. The disposable absorbent article (10) of claim 3 wherein the at least one pleat (42) is adhered in the front (20) and rear (22) waistband regions.

5. The disposable absorbent article of any preceding claim wherein the liner (30) extends transversely to each side margin (16a,16b).

6. The disposable absorbent article (10) of any preceding claim wherein the backsheet (40) has an hourglass shape with the narrow portion located in the intermediate region (24).

7. The disposable absorbent article (10) of any preceding claim further comprising filler material located within the pleat (42).

8. The disposable absorbent article (10) of any preceding claim further comprising at least one pleat (36) formed on the liner (30).

9. The disposable absorbent article (10) of any preceding claim wherein the backsheet (40) includes a plurality of pleats (42).

10. The disposable absorbent article (10) of any preceding claim wherein each of the pleats (42) extend in a direction substantially parallel to the longitudinal axis (14).

11. The disposable absorbent article (10) of any preceding claim further comprising at least two containment flaps (19), one of the flaps being disposed between each of the pleats (42) and the adjacent side margin (16a,16b).

12. The disposable absorbent article (10) of any preceding claim wherein the liner (30) completely surrounds the absorbent core (50).

13. The disposable absorbent article (10) of claim 12 wherein the liner (30) is associated with the backsheet (40) only in the intermediate area (24).

14. The disposable absorbent article (10) of any preceding claim wherein the liner (30) is joined to the backsheet (40) between the at least one pleat (42) and the longitudinal axis (14).

15. The disposable absorbent article (10) of any preceding claim wherein the at least one pleat (42) is defined by a first pleat layer formed in the liner (30) and a second pleat layer formed in the backsheet (40), wherein the first pleat layer is disposed outside the second pleat layer in an initial unexpanded condition and the first pleat layer is disposed within the second pleat layer after expansion of the pleat (42).

16. The disposable absorbent article (10) of claim 15 wherein the first and second pleat layers are disposed transversely outboard of a longitudinal edge of the absorbent core (50) in the intermediate region (24).

17. The disposable absorbent article (10) of any preceding claim wherein the liquid pervious liner (30) defines an exposed inner surface (32) of the absorbent article;
the liquid impervious backsheet (40) has a greater lateral width than the liner (30);
the absorbent core (50) has longitudinally extending outer edges disposed inwardly of the side margins (16a,16b); and
each of the pleats (42) is disposed transversely outboard of the liner (20).

18. The disposable absorbent article (10) of claim 17 wherein the liner (30) completely encircles the absorbent core (50) in at least a portion of the intermediate region (24).

19. The disposable absorbent article (10) of claim 17 wherein the liner (30) is attached to the backsheet (40) at a location between the pleats (42).

## Patentansprüche

1. Wegwerfbarer absorbierender Artikel (10) mit einer Längsachse (14) und einer Querachse (12), einem vorderen Hüftbund-Bereich (20), einem hinteren Hüftbund-Bereich (22) und einem Zwischenbereich (24), der den vorderen und den hinteren Hüftbund-Bereich miteinander verbindet und zwischen einander seitlich gegenüberliegenden Seitenrändern (16a, 16b) angeordnet ist, wobei der Artikel des Weiteren umfasst:
a. eine flüssigkeitsdurchlässige Auskleidung (30);
b. eine flüssigkeitsundurchlässige Trägerbahn (40);
c. einen absorbierenden Kern (50), der zwischen der Auskleidung und der Trägerbahn angeordnet ist, wobei der absorbierende Kern einen durchgehenden Querschnitt sowie eine erste (52a) und eine zweite (52b) seitliche Längskante aufweist;
**gekennzeichnet durch**:
d. wenigstens eine Falte (42), die in der Trägerbahn angrenzend an jeden seitlichen Rand gebildet ist, wobei die Falte in dem Zwischenbereich (24) nicht anhaftet, so dass sich die Trägerbahn (40) in dem Zwischenbereich (22) vom Körper des Trägers weg ausdehnen kann, wobei die Falte, wenn sich die Falte ausdehnt, ein ausgedehntes Volumen bildet, das größer ist als das Volumen, wenn die Falte nicht ausgedehnt ist, und die erste und die zweite seitliche Längskante des absorbierenden Kerns sich, wenn sich die Falte ausdehnt, nicht über den innersten Bereich der Falte, die in der Trägerbahn gebildet ist, hinaus erstreckt, und dass der wegwerfbare, absorbierende Artikel (10) weiterhin eine Einschlusslasche (19), die von jedem seitlichen Rand nach innen beabstandet ist, und ein elastisches Element (18), das mit jedem seitlichen Rand (16a, 16b) verbunden ist, um elastische Beinöffnungen zu bilden, umfasst.

2. Wegwerfbarer absorbierender Artikel (10) nach Anspruch 1, wobei sich die wenigstens eine Falte (42) in einer Richtung im Wesentlichen parallel zu der Längsachse (14) erstreckt.

3. Wegwerfbarer absorbierender Artikel (10) nach Anspruch 2, wobei sich die wenigstens eine Falte (42) im Wesentlichen von dem vorderen Hüftbund-Bereich (20) zu dem hinteren Hüftbund-Bereich (22) erstreckt.

4. Wegwerfbarer absorbierender Artikel (10) nach Anspruch 3, wobei die wenigstens eine Falte (42) in dem vorderen (20) und dem hinteren (22) Hüftbund-Bereich anhaftet.

5. Wegwerfbarer absorbierender Artikel (10) nach einem der vorangehenden Ansprüche, wobei sich die Auskleidung (30) quer zu jedem seitlichen Rand (16a, 16b) erstreckt.

6. Wegwerfbarer absorbierender Artikel (10) nach einem der vorangehenden Ansprüche, wobei die Trägerbahn (40) eine Sanduhrform hat und sich der schmale Abschnitt in dem Zwischenbereich (24) befindet.

7. Wegwerfbarer absorbierender Artikel (10) nach einem der vorangehenden Ansprüche, der des Weiteren ein Füllmaterial umfasst, das sich in der Falte (42) befindet.

8. Wegwerfbarer absorbierender Artikel (10) nach einem der vorangehenden Ansprüche, der des Weiteren wenigstens eine Falte (36) umfasst, die an der Auskleidung (30) gebildet ist.

9. Wegwerfbarer absorbierender Artikel (10) nach einem der vorangehenden Ansprüche, wobei die Trägerbahn (40) eine Vielzahl von Falten (42) enthält.

10. Wegwerfbarer absorbierender Artikel (10) nach einem der vorangehenden Ansprüche, wobei sich jede der Falten (42) in einer Richtung im Wesentlichen parallel zu der Längsachse (14) erstreckt.

11. Wegwerfbarer absorbierender Artikel (10) nach einem der vorangehenden Ansprüche, der des Weiteren wenigstens zwei Einschlusslaschen (19) umfasst, wobei eine der Laschen zwischen jeder der Falten (42) und dem angrenzenden seitlichen Rand (16a, 16b) angeordnet ist.

12. Wegwerfbarer absorbierender Artikel (10) nach einem der vorangehenden Ansprüche, wobei die Auskleidung (30) den absorbierenden Kern (50) vollständig umgibt.

13. Wegwerfbarer absorbierender Artikel (10) nach Anspruch 12, wobei die Auskleidung (30) mit der Trägerbahn (40) nur in dem Zwischenbereich (24) verbunden ist.

14. Wegwerfbarer absorbierender Artikel (10) nach einem der vorangehenden Ansprüche, wobei die Auskleidung (30) mit der Trägerbahn (40) zwischen der wenigstens einen Falte (42) und der Längsachse (14) verbunden ist.

15. Wegwerfbarer absorbierender Artikel (10) nach einem der vorangehenden Ansprüche, wobei die wenigstens eine Falte (42) durch wenigstens eine Faltenschicht, die in der Auskleidung (30) ausgebildet ist, und einer zweiten Faltenschicht, die in der Trägerbahn (40) ausgebildet ist, gebildet wird, wobei die erste Faltenschicht in einem anfänglichen, nicht ausgedehnten Zustand außerhalb der zweiten Faltenschicht angeordnet ist und die erste Faltenschicht nach dem Ausdehnen der Falte (42) in der zweiten Faltenschicht angeordnet ist.

16. Wegwerfbarer absorbierender Artikel (10) nach Anspruch 15, wobei die erste und zweite Faltenschicht quer außerhalb einer Längskante des absorbierenden Kerns (50) in dem Zwischenbereich (24) angeordnet sind.

17. Wegwerfbarer absorbierender Artikel (10) nach einem der vorangehenden Ansprüche, wobei die flüssigkeitsdurchlässige Auskleidung (30) eine freiliegende Innenfläche (32) des absorbierenden Artikels bildet,
wobei die flüssigkeitsundurchlässige Trägerbahn (40) eine größere seitliche Breite hat als die Auskleidung (30),
der absorbierende Kern (50) in Längsrichtung verlaufende Außenkanten aufweist, die innerhalb der seitlichen Ränder (16a, 16b) angeordnet sind; und
jede der Falten (42) quer außerhalb der Auskleidung (20) angeordnet ist.

18. Wegwerfbarer absorbierender Artikel (10) nach Anspruch 17, wobei die Auskleidung (30) den absorbierenden Kern (50) in wenigstens einem Teil des Zwischenbereiches (24) vollständig umschließt.

19. Wegwerfbarer absorbierender Artikel (10) nach Anspruch 17, wobei die Auskleidung (30) an der Trägerbahn (40) an einer Position zwischen den Falten (42) angebracht ist.

## Revendications

1. Article absorbant jetable (10) ayant un axe longitudinal (14) et un axe transversal (12), une région de ceinture avant (20), une région de ceinture arrière (22), et une région intermédiaire (24) interconnectant les régions de ceinture avant et arrière et disposée entre des marges latérales (16a,16b) transversalement opposées, l'article comprenant en outre:
a. une doublure (30) perméable aux liquides;
b. une feuille support (40) imperméable aux liquides ;
c. un noyau absorbant (50) disposé entre la doublure et la feuille support, le noyau absorbant ayant une section transversale ininterrompue et des premier (52a) et second (52b) bords latéraux, **caractérisé par**
d. au moins un pli (42) formé dans la feuille support adjacent à chaque marge latérale, ledit pli n'étant pas collé à la région intermédiaire (24), de telle sorte qu'il permet à la feuille support (40) de se dilater à l'écart du corps du porteur dans la région intermédiaire (24), le pli définissant un volume dilaté lorsque le pli se dilate, volume dilaté qui est supérieur au volume existant lorsque le pli n'est pas dilaté et, lorsque le pli se dilate, les premier et second bords latéraux transversaux du noyau absorbant ne s'étendent pas au-delà de la portion la plus interne du pli formé dans la feuille support, et en ce que l'article absorbant jetable (10) comprend en outre un volet de retenue (19) à l'écart, vers l'intérieur, de chaque marge latérale, et un élément élastique (18) connecté à chaque marge latérale (16a,16b) pour réaliser des ouvertures de jambe élastiquées.

2. Article absorbant jetable (10) selon la revendication 1, dans lequel ledit au moins un pli (42) s'étend dans une direction sensiblement parallèle à l'axe longitudinal (14).

3. Article absorbant jetable (10) selon la revendication 2, dans lequel ledit au moins un pli (42) s'étend sensiblement depuis la région de ceinture avant (20) jusqu'à la région de ceinture arrière (22).

4. Article absorbant jetable (10) selon la revendication 3, dans lequel ledit au moins un pli (42) est collé dans les régions de ceinture avant (20) et arrière (22).

5. Article absorbant jetable (10) selon l'une quelconque des revendications précédentes, dans lequel la doublure (30) s'étend transversalement jusqu'à chaque marge latérale (16a,16b).

6. Article absorbant jetable (10) selon l'une quelconque des revendications précédentes, dans lequel la feuille support (40) a une forme en sablier dont la portion étroite est située dans la région intermédiaire (24).

7. Article absorbant jetable (10) selon l'une quelconque des revendications précédentes, comprenant en outre un matériau formant charge situé au sein du pli (42).

8. Article absorbant jetable (10) selon l'une quelconque des revendications précédentes, comprenant en outre au moins un pli (36) formé sur la doublure (30).

9. Article absorbant jetable (10) selon l'une quelconque des revendications précédentes, dans lequel la feuille support (40) comprend une pluralité de plis (42).

10. Article absorbant jetable (10) selon l'une quelconque des revendications précédentes, dans lequel chacun des plis (42) s'étend dans une direction sensiblement parallèle à l'axe longitudinal (14).

11. Article absorbant jetable (10) selon l'une quelconque des revendications précédentes, comprenant en outre au moins deux volets de retenue (19), l'un des volets étant disposé entre chacun des plis (42) et la marge latérale (16a,16b) adjacente.

12. Article absorbant jetable (10) selon l'une quelconque des revendications précédentes, dans lequel la doublure (30) entoure complètement le noyau absorbant (50).

13. Article absorbant jetable (10) selon la revendication 12, dans lequel la doublure (30) est associée à la feuille support (40) uniquement dans la région intermédiaire (24).

14. Article absorbant jetable (10) selon l'une quelconque des revendications précédentes, dans lequel la doublure (30) est réunie à la feuille support (40) entre ledit au moins un pli (42) et l'axe longitudinal (14).

15. Article absorbant jetable (10) selon l'une quelconque des revendications précédentes, dans lequel ledit au moins un pli (42) est défini par une première couche de pli formée dans la doublure (30) et une seconde couche de pli formée dans la feuille support (40), ladite première couche de pli étant disposée à l'extérieur de ladite seconde couche de pli dans un état initial non dilaté et la première couche de pli étant disposée au sein de la seconde couche de pli après la dilatation du pli (42).

16. Article absorbant jetable (10) selon la revendication 15, dans lequel lesdites première et seconde couches de pli sont disposées transversalement au-delà d'un bord longitudinal du noyau absorbant (50) dans la région intermédiaire (24).

17. Article absorbant jetable (10) selon l'une quelconque des revendications précédentes, dans lequel la doublure (30) perméable aux liquides définit une surface intérieure exposée (32) dudit article absorbant;
la feuille support (40) imperméable aux liquides a une largeur transversale supérieure à celle de la doublure (30) ;
le noyau absorbant (50) a des bords extérieurs s'étendant longitudinalement disposés en deçà des marges latérales (16a,16b) ; et
chacun des plis (42) est disposé transversalement au-delà de la doublure (30).

18. Article absorbant jetable (10) selon la revendication 17, dans lequel la doublure (30) encercle totalement le noyau absorbant (50) dans au moins une portion de la région intermédiaire (24).

19. Article absorbant jetable (10) selon la revendication 17, dans lequel la doublure (30) est fixée à la feuille support (40) en un emplacement situé entre les plis (42).
